# EUROPEAN PATENT SPECIFICATION

(11) **EP 4 389 030 B1**
(45) Date of publication and mention of the grant of the patent: **23.07.2025**
(21) Application number: 23150675.9
(22) Date of filing: 09.01.2023
(51) Int. Cl.: A61B 17/66, A61B 17/88, B25B 21/00, A61B 90/00, B25B 23/00

(54) **TOOL FOR EXTERNAL FIXATION STRUT ADJUSTMENT, ADJUSTABLE EXTERNAL FIXATION STRUT, AND KIT COMPRISING SAID FIXATOR STRUT AND SAID TOOL**
WERKZEUG ZUR EXTERNEN FIXATIONSSTREBENEINSTELLUNG, VERSTELLBARE EXTERNE FIXATIONSSTREBE UND KIT MIT DER FIXATIONSSTREBE UND DEM WERKZEUG
OUTIL POUR LE RÉGLAGE D'UNE ENTRETOISE DE FIXATION EXTERNE, ENTRETOISE DE FIXATION EXTERNE RÉGLABLE, ET KIT COMPRENANT LADITE ENTRETOISE DE FIXATION ET LEDIT OUTIL

(30) Priority: 23.12.2022 US 202263477095 P
(43) Date of publication of application: 26.06.2024
(73) Proprietor: Orthofix S.r.l., 37012 Bussolengo (VR) (IT); Texas Scottish Rite Hospital For Children, Dallas, TX 75219 (US)
(72) Inventor: OTTOBONI, Andrea, 45020 Giacciano con Baruchella (RO) (IT); DONNICI, Mario, 37012 Bussolengo (VR) (IT); MISTRETTA, Paolo, 37121 Verona (IT); ROSS, John David, Ovilla, TX 75154-5832 (US); STANDEFER, Karen Divita, Flower Mound, TX 75022 (US); SAMCHUKOV, Mikhail, Coppell, TX 75019 (US); CHERKASHIN, Alexander, Flower Mound, TX 75022 (US)
(74) Representative: Botti & Ferrari S.p.A.

(56) References cited:
- WO-A1-2009/105479
- WO-A1-2022/254320
- US-A1- 2002 010 465
- US-A1- 2008 051 779

## Description

### Technical field

The present disclosure relates to the general field of external fixation, and more specifically, to the connection between external fixator struts and dedicated tools employed for the incremental or decremental adjustment of such struts, during preoperative, intraoperative and postoperative periods.

### Background

Without limiting the scope of the present disclosure, its background is herein described in connection with external fixation devices and related tools for the adjustment of struts or other connection rods thereof.

Generally speaking, external fixation devices are commonly used in a variety of surgical procedures including limb fracture fixation, lengthening and deformity correction. The process involves the application of a rigid framework. This can be a circular system, such as a hexapod system and/or a circular system with a plurality of struts. Such systems usually comprise several rings or arches that are placed externally around the limb and attached to bone segments using wires and half pins inserted into the bone segments and connected to the related section of the external rigid framework. Also, monolateral systems, such as monolateral rails or monolateral unibody, can be employed.

Rings of the rigid framework located opposite to one another are interconnected by either threaded and/or telescopic struts directly or in conjunction with uniplanar or multiplanar hinges, which allows to adjust position of the rings relative to each other longitudinally, rotationally, horizontally or angularly over a period of time.

For example, in limb lengthening, the bone is surgically divided into two segments and wires and half pins are inserted into bone segments above and below the surgical bone cut and attached to rings of a rigid framework interconnected by struts or telescopic connection struts.

For limb lengthening, the opposite rings are preferably interconnected directly by at least three or four threaded or telescopic struts that are regularly adjusted in length and allowed for gradual separation of bone segments longitudinally.

The rigid framework is used to gradually push the two bone segments apart longitudinally over a period of time (for instance, one millimeter a day). This allows the new bone to gradually form in the gap between bone segments created by this distraction technique. Once the desired amount of lengthening is achieved (e.g., 5-6 cm), the external apparatus is stabilized into a fixed position and left on the bone segments until complete mineralization of the newly formed bone (e.g., 3-6 months, depending on the nature of pathology and/or amount of lengthening).

Similarly, in deformity correction, the bone is surgically divided (usually at the apex of the deformity) into two segments and wires and half pins are inserted into bone segments above and below the surgical bone cut and attached to rings of a rigid framework. In this case also opposite rings of the rigid framework are connected together by threaded struts with attached hinges and angular distractor that is used to gradually push the two bone segments apart angularly over a period of time.

One common fixation device is a circular metal structure known as the Ilizarov apparatus. The Ilizarov apparatus, when used for limb lengthening or deformity correction, consists of several rings or arches that are placed externally around the limb and attached to surgically separated bone segments using wires and half pins. For angular deformity correction, the opposite rings of the Ilizarov apparatus are connected by a pair of hinges that provide an axis of rotation for bone segments and an angular distractor that gradually pushes two rings and associated bone segments apart.

Another common external fixation device is known as Taylor Spatial Frame, which is a hexapod type external fixation device based on a so-called Stewart platform but shares many components and features of the Ilizarov apparatus.

The Taylor Spatial Frame comprises two external fixation rings attached to bone segments by wires and half pins and connected together by five or six telescopic struts with multi-planar hinges located at both ends of the struts. Each strut may be lengthened or shortened as necessary to either pull two interconnected ring segments towards each other or push them apart.

Other examples of fixation devices of this kind are commercially known as TrueLok and Sheffield.

Adjustment of strut length allows manipulating with bone segments acutely or gradually in several axes to perform limb lengthening and correct angular, translational and rotational deformities simultaneously.

The amount of daily strut length adjustment is usually calculated by a dedicated software. Once the apparatus is attached to the bone segments, numerous parameters such as deformity parameters, frame parameters, and mounting parameters and so forth are entered into the software to characterize one ring position relative to another ring and position of bone segments relative to each other and to the rings. After calculation of the total amount of each strut length adjustment, the software provides a tabled instruction ("prescription") on the amount of each strut length adjustment that should be achieved per each increment, including a number identifying the single strut, the amount of adjustment required, and the time scheduled for such and adjustment. In most cases of deformity correction, the struts are adjusted in different directions (shortening/lengthening) and in different amounts.

Since the prescription requires several adjustments over time, usually up to four adjustments per day, most of the time these regulations cannot be performed by the surgeon or by a dedicated practitioner. The task of following the prescription is thus entrusted to the patient or to one of their relatives, which do so by turning an adjustment knob on the struts or by turning the nuts of the threaded rods with a wrench.

This way of strut length adjustment is time consuming (e.g., due to loosening and retightening of the threaded rod nuts before and after each adjustment), does not provide precise length adjustment (e.g., due to difficulty to monitor small amounts of adjustments) and creates overall frame instability during adjustments (e.g., due to dimensional clearance between connection elements).

Furthermore, the prescription for length adjustments can be complicated, and human errors are prone to occur during the course of a complicated prescription. Even though the patient is invited to verify compliance between prescription and frame status by inspecting the strut length, an error can well remain unnoticed, due to a negligent check or a total lack thereof.

Additionally, it must be considered that the feedback to the surgeon depends on the patient, who is demanded to communicate the adjustments made on the struts, usually by uploading the information on a dedicated portal. Once again, carelessness by the patient may result in incorrect or incomplete information given to the surgeon.

It is easy to understand that errors in carrying out the prescription, particularly if they are not promptly identified by the surgeon, may result in detrimental effects on the final outcome of the correction process.

To alleviate the aforementioned drawbacks, in recent year a programmable tool has been proposed for incrementally adjusting the length of the external fixator strut. The tool, described for instance in prior art application WO 2009/105479 in the name of Texas Scottish Rite Hospital for Children, is designed as an electric wrench meant to engage with the adjustment mechanism of the external fixator struts. The tool has an internal memory for storing the adjustment parameters of the prescription and is set to automatically adjust each of the struts according to said parameters.

Even though it provides numerous advantages over the previously discussed prior art, the programmable tool has however remaining drawbacks, in particularly relating to the ease of use of the device.

Indeed, in order to correctly adjust the length parameters of the struts according to the memorized prescription, it is advisable to feed the tool with real-time measurements of the struts' length. If the tool were to implement the length increments or decrements according to the prescription without feedback, the adjustment process could easily get flawed due to incremental errors, and any erroneous action by the patient - for instance coupling the tool to the wrong strut when applying the prescription - would probably disrupt the correct application of the adjustment plan over time.

Therefore, the programmable tool is preferably provided with means to measure the strut, for instance a digital ruler which is meant to be coupled to the opposite ends thereof. However, such measurement means add to the complexity of the device, and more importantly they require a positive action by the patient, thus making the process of applying the prescription more difficult and prone to human error.

Devices according to the prior art are disclosed in documents US 2008/051779 A1 and US 2002/010465 A1.

The technical problem underlining the present invention is that of providing a new system for adjusting external fixation that solves, or at least alleviates, the drawbacks identified with respect to the prior art.

A main aim of the improved system of the present disclosure is that of enhancing the automation of the adjustment process, without burdening to the user with additional tasks such as identification of the strut number and external measurement of the strut length while maintaining the design of the system streamlined and cost-effective.

### Summary of the Invention

The solution idea at the basis of the present disclosure is that of providing an inner sensor within the external fixation strut to measure a length of said strut, said sensor communicating directly with a programmable tool for lengthening/ shortening the strut via a data port.

According to such a solution idea, the technical problem underlying the invention is solved by an external fixation strut comprising:
an elongated body comprising at least a first shaft and a second shaft moveable with respect to each other to modify the length of said elongated body;
at least a sensor integrated in the external fixation strut and adapted to perform at least a measurement which is indicative of the length of the elongated body;
wherein said sensor communicates with a programmable tool through a port which is engageable by said programmable tool.

In a preferred embodiment, said data port is also used for powering the sensor.

This provides the advantage that the external fixation strut does not need an integrated power supply, such as a battery: the external power supply is enough to provide the measurement when needed, namely during the operation of the programmable tool.

In a preferred embodiment, the sensor is an absolute sensor as opposed to a relative sensor, i.e. it returns a position in absolute terms instead of returning increments and decrements at each operation of the programmable tool.

Said sensor uses either an inductive or a capacitive technology to determine the position of a movable element. A best mode of the invention employs inductive technology.

The movable element which is sensed is an end-piece of the second shaft and/or a metering cursor which translates with said end-piece with respect to the second shaft.

The end-piece is slidable within a tubular body of the first shaft. In particular, the end-piece may be a translatable nut which is rotatingly connected to an internal free end of a threaded shank of the second shaft.

In case of the sensor using inductive technology, the sensed element is preferably a metering cursor which is attached to the end-piece.

In an embodiment, such a metering cursor is external to tubular body of the first shaft, emerging from a longitudinal slit of said tubular body. Such a solution gives better sensitivity because the measuring is not influenced by the surrounding tubular body.

In a different embodiment, at least a predominant part of said metering cursor is housed within the tubular body of the first shaft. Such an embodiment is preferred because, even though a fine tuning of the sensor is needed to cancel out the noise due to the surrounding body, the internal metering cursor is less prone to breakage and does not impede visual reading on external indexes.

Preferably, said metering cursor is at least partially housed within a longitudinal slit of the tubular body of the first shaft, said longitudinal slit extending in a longitudinal direction of the strut.

Preferably, said metering cursor engages with the end-piece via a groove and tooth arrangement which extends in a transverse direction orthogonal to the longitudinal direction of the strut, so that the metering cursor can be mounted by inserting it through the longitudinal slit.

Such a solution is particularly expedient since it simplifies the manufacturing of the external fixation strut.

The sensor comprises a sensing strip arranged within the tubular body of the first shaft.

The metering cursor, if present, slides over said sensing strip.

The sensing strip is a flexible printed circuit board, and it is attached on top of a rigid support plate.

The use of a rigid support plate simplifies the manufacturing of the device, since the sensing strip can be attached to the plate prior to the insertion in the tubular body.

The rigid support plate is made of ferromagnetic material, such as ferrite. This shields at least partially the sensing strip from external noise.

As mentioned above, a longitudinal slit is provided along the tubular body of the first shaft. This way, the sensing strip and the rigid support plate can be inserted through said longitudinal slit in a mounting phase, making much easier to manufacture the device.

The longitudinal slit also makes it possible to visually detect the position of the metering cursor, which can be used as a visual index for the relative position of the second shaft with respect to the first shaft.

Preferably, the metering cursor comprises a copper layer which is fixed on the underside of the cursor. The copper layer is meant to amplify the antenna gain, thereby increasing the sensitivity of the metering device. The copper layer is most preferably gold-plated to avoid corrosion issues.

Preferably, second shaft comprises a casing having a tubular body and a protruding portion extending beyond the diameter of said tubular body, said protruding portion housing an electronic board for sensor powering and data transmission which is not aligned with the sensing strip, the electronic board being connected to the sensing strip through a flexible signal line.

Preferably, the data port may be provided in connection with a mechanical torque transmission port which is provided to engage the programmable tool to external fixation strut.

In preferred embodiments, the external fixation strut further comprises a rotatable adjustment mechanism for moving the second shaft with respect to the first shaft thus modifying the length of the elongated body; a second coupling portion of the rotatable adjustment mechanism adapted to releasably engage with a first coupling portion of the programmable tool to enable torque transmission; wherein said port is defined by a second connecting portion of the rotatable adjustment mechanism which is in electrical communication with a first connecting portion of the programmable tool when the first coupling portion is engaged with the second coupling portion, and which enables transmission of data from the sensor to the programmable tool.

The aforementioned technical problem is also solved by a medical kit comprising both the external fixation strut previously discussed and a programmable tool operable to adjust said external fixation strut and comprising:
an output shaft;
a motor operable to rotate said output shaft;
a first coupling portion solidly attached to said output shaft and adapted to releasably engage with a corresponding second coupling portion of a rotatable adjustment mechanism of the external fixation strut, thus enabling torque transmission from the motor to the rotatable adjustment mechanism;
a controller configured for driving said motor according to a prescription comprising instructions for adjusting the external fixation strut;
a first connecting portion in electrical communication with said controller and adapted to electrically connect with a second connecting portion of the rotatable adjustment mechanism when the first coupling portion is engaged with the second coupling portion, thus enabling data transmission from said sensor to the controller.

### Brief description of the drawings

For a more complete understanding of the features and advantages of the present disclosure, reference is now made to the detailed description of four different struts embodiments of the invention along with the accompanying figures and in which:
FIG. 1 is a side view of a first embodiment of an external fixation strut according to the present disclosure;
FIG. 2 is a cross-sectional view of an external fixation strut, taken along the cutting plane referenced with A-A in FIG. 1;
FIG. 3 is a cross-sectional view of an external fixation strut, taken along the cutting plane referenced with B-B in FIG. 1;
FIG. 4 is a perspective view of a second embodiment of an external fixation strut according to the present disclosure;
FIG. 5 is a detail of the perspective view of FIG. 4;
FIG. 6 is a cross-sectional view of the external fixation strut of FIG.4, taken along a mid-section of the extractable tube;
FIG. 7 is a side view of a third embodiment of an external fixation strut according to the present disclosure;
FIG. 8 is a cross-sectional view of an external fixation strut, taken along the cutting plane referenced with C-C in FIG. 7;
FIG. 9 is a perspective view of an embodiment of an external fixation strut according to the present disclosure;
FIG. 10 is a side view of a fourth embodiment of an external fixation strut according to the present disclosure;
FIG. 11 is a cross-sectional view of an external fixation strut, taken along the cutting plane referenced with D-D in FIG. 10;
FIG. 12 is a cross-sectional view of an external fixation strut, taken along the cutting plane referenced with E-E in FIG. 10;
FIG. 13 is a side view of the fourth embodiment of an external fixation strut according to the present disclosure;
FIG. 14 is a cross-sectional view of an external fixation strut, taken along the cutting plane referenced with F-F in FIG. 10;
FIG. 15 is a perspective view of a fourth embodiment of an external fixation strut according to the present disclosure;
FIG. 16 is an enlarged detail of the area referenced with G in FIG. 14;
FIG. 17 is an enlarged detail of the area referenced with H in FIG. 14;
FIG. 18 is a top view of an embodiment of a programmable tool according to the present disclosure;
FIG 19 is a side view of an embodiment of an external fixation strut next to an embodiment of a programmable tool, according to the present disclosure;
FIG. 20 is a cross-sectional view of an external fixation strut next to a programmable tool, taken along the cutting plane referenced with I-I in FIG. 19;
FIG 21 is a side view of an embodiment of an external fixation strut next to an embodiment of a programmable tool, according to the present disclosure;
FIG. 22 is a cross-sectional view of an external fixation strut next to a programmable tool, taken along the cutting plane referenced with J-J in FIG. 21;
FIG. 23 is an enlarged detail of the area referenced with K in FIG. 22;
FIG. 24 is a top part of a flow chart showing operation of a programmable tool according to the present disclosure;
FIG 25 is the bottom part of the flow chart of FIG. 24.

### Detailed description

While the making and using of various embodiments of the present disclosure are discussed in detail below, it should be appreciated that the present disclosure provides many applicable inventive concepts that can be embodied in a wide variety of specific contexts. The specific embodiments discussed herein are merely illustrative of specific ways to make and use the disclosure and do not delimit the scope of the disclosure.

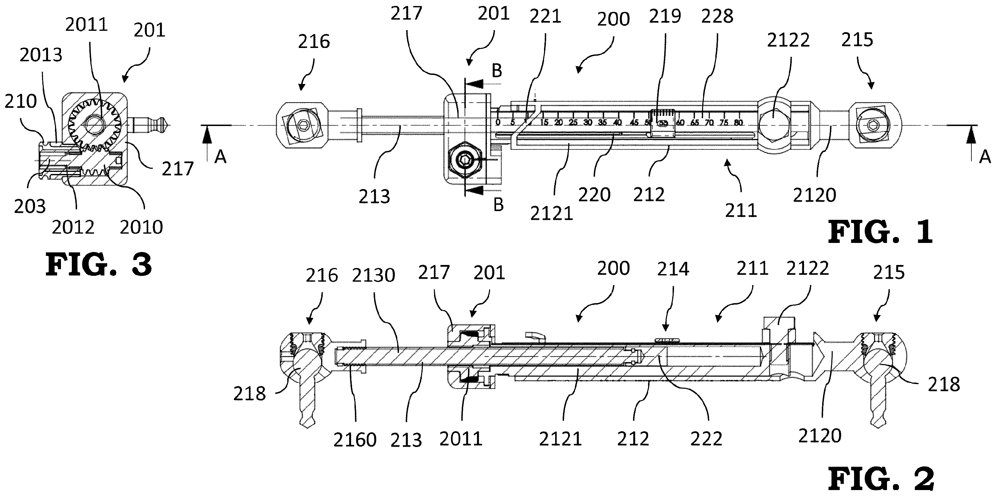

**Figure 1** shows a schematic side view of an improved external fixation strut 200 according to the present disclosure having an elongated body 211 with opposite ends 215, 216 configured to be attached to a respective fixation ring or arch of an external fixator, preferably of the hexapod type.

The rings of the body of the external fixator, which can be fixed to bone sites via half pins or wires, are known per se and not depicted in the enclosed figures.

The elongated body 211 is formed by a first hollow tubular shaft 212 in which a second tubular shaft 213 is slidably hosted. The first shaft 212 is in turn composed of a hollow main body 2120, which culminates with the first end 215, and a sliding body 2121 which slides within the main body 2120 and houses the second tubular shaft 213.

The relationship between the two bodies of the first 212 shaft and between the first shaft and the second shaft 213 confers a telescopic configuration to the ensemble of the strut 200, so that the elongated body 211 may be adjusted in its length according to the needs of keeping the interconnected rings in a predetermined relative spatial relationship.

In particular, the relationship between the main body 2120 and the sliding body 2121, which defines the length of the first shaft 212, is varied in a first regulation of the length between the two attachment points of the strut 200, while the relationship between the first shaft 212 and the second shaft 213 is varied to finely adjust the strut length, in particular in a postoperative follow-up.

The main body 2120 and the sliding body 2121 are locked together by a locking screw 2122, which is released for manual adjustment of the relative position of the two elements.

On the contrary, the position of the second shaft 213 with respect to the first shaft 212 is adjusted through a rotatable adjustment mechanism 201 contained in a casing 217 of the sliding body 2121 of the first shaft 212. The rotatable adjustment mechanism will be described in greater detail with reference to Figure 3.

The casing 217 has a tubular portion 217a, which can be slid inside a sleeved portion of the main body 2120, and at the end thereof a protruding portion 217b which extends beyond the diameter of the tubular portion 217a. The rotatable adjustment mechanism 201 is conveniently housed in the protruding portion 217b.

An external metering cursor 219, which is solidly attached to an inner end of the second shaft 213 through a leg traversing a longitudinal slit 220 of the sliding body 2121, slides along a graduated scale 228 integral with the casing 217 and makes it possible to visually assess the relative position of the second shaft 213 with respect to the first 212.

To make the graduated scale 228 visible to the user, the frame of the hollow main body 2120 has an open side. A transverse bridge 221 of the frame is provided at an end of said open side. The bridge 221 is visible against the background of the graduated scale 228, making it possible to visually assess the relative position of the sliding body 2121 with respect to the main body 2120, i.e. the length of the first shaft 212.

**Figure** 2 shows a cross-sectional view of the device of figure 1.

At the two ends 215, 216 of the strut 200, ball-joints 218 are provided which makes it possible to articulate the strut 200 with respect to a fixation ring or arch to which it is attached.

The rotation imparted through the rotatable adjustment mechanism 201 defines, through a threaded connection, a displacement of the second shaft 213 with respect to the first shaft 212, so that the second end 216 is pulled away or dragged towards the first end 215.

In particular, the second shaft 213 has an intermediate shank 2130 which is externally threaded. The intermediate shank 2130 is rotatably inserted within a sleeve 2160 of the respective end 216 of the strut 200 on one side, and within a slidable end-piece 222 on the other side.

**Figure 3** shows a cross-sectional view of the above-mentioned rotatable adjustment mechanism 201.

The rotatable adjustment mechanism 201 comprises a worm gear mechanism having a worm screw 2010 meshing with a worm gear 2011, the latter being coaxial with the second shaft 213 and threadingly engaged with it. Therefore, a rotation of the worm screw 2010 determines a corresponding rotation of the worm gear 2011 which, in turn, drives the second shaft 213 into translation with respect to the first shaft 212.

The worm screw 2010 has a worm screw shaft 2012 which is orthogonal to the axis of the elongated body 211.

A protruding head 2013, which protrudes from a lateral wall of the casing 217, rotatingly houses said screw shaft 2010 and, as will be further discussed in the following, defines a second coupling portion 203 for the attachment of a corresponding coupling portion of a wrench tool.

The protruding head 2013 further defines an outer sleeve surrounding the second coupling portion 203. In the depicted embodiment, the outer sleeve has an inner circular profile and an outer hexagonal profile, the latter comprising an external groove 210 for attachment of a locking mechanism of an adjustment tool.

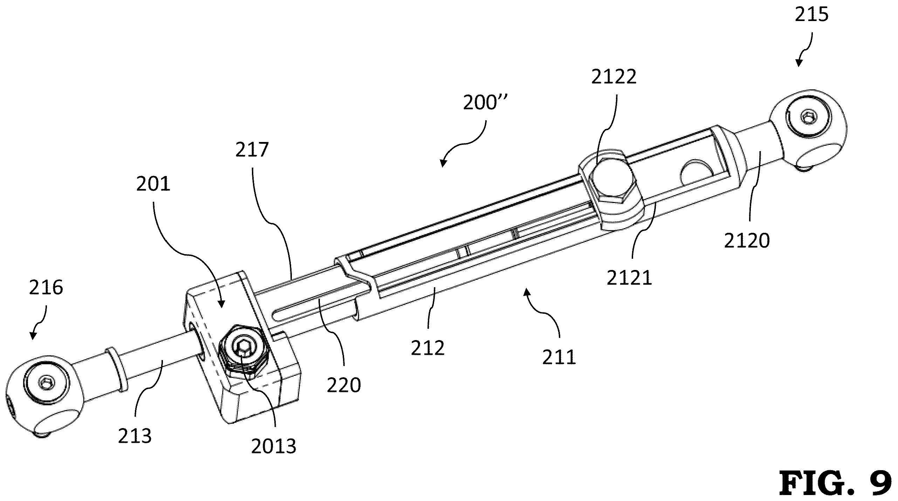

Further details of the of the attachment will be discussed with reference to the embodiment which is depicted in Figure 9 and which features an identical attachment portion.

A position sensor 214, which is visible in previously discussed Figure 2, is provided within the first shaft 212 to sense the position of the metering cursor 219. In this embodiment of the strut, the position sensor is based on inductive technology. The position sensor is glued on the external surface. The sensor is also positioned within the tubular element.

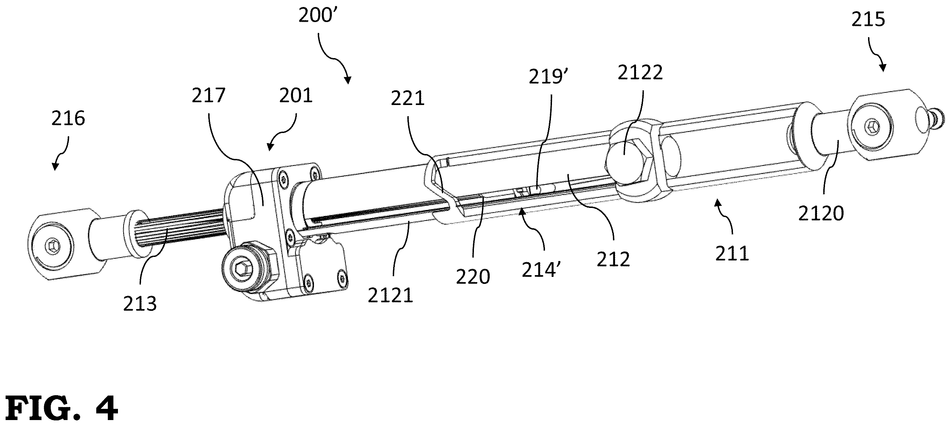

**Figure 4** refers to an alternative embodiment of an external fixation strut 200', which features an improved version of the inductive position sensor 214'.

In such an alternative embodiment, the external fixation strut 200' essentially has the same components and main features of the previous embodiments, which is previously described. Such components and features are therefore indicated in the figures by reference numerals previously used, and they are not described anew in the following paragraphs.

The inductive position sensor 214' differs from the previously described position sensor 214 mainly in that the metering cursor 219', the position of which is detected, is kept within the body of the first shaft 212: in particular, within the tubular element defined by the sliding body 2121.

The internal metering cursor 219' is preferable since it does not interfere with the readings on the external scales, and it greatly reduces the risk of damages from handling.

On the other hand, the surrounding sliding body 2121 heavily interferes with the reading of the position of the internal metering cursor 219', and the position sensor 214' needs to be accurately tuned in order to cancel out the noise.

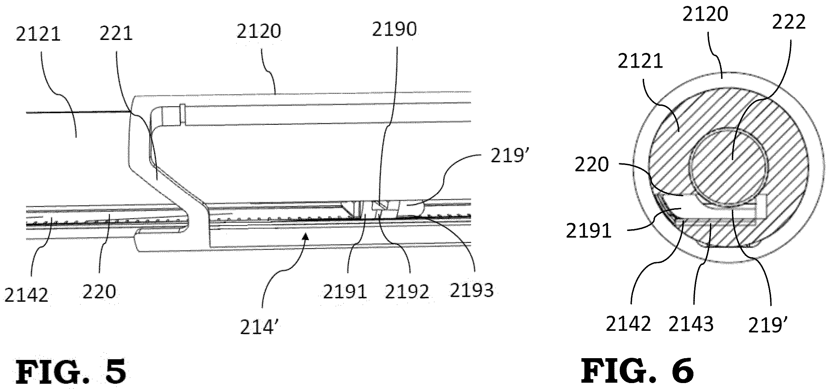

**Figure 5** shows an enlarged detail of the inductive position sensor 214' from previous Figure 4.The internal metering cursor 219' has a sled-shaped body, featuring an upper transverse groove 2190 for insertion of a corresponding tooth provided on the bottom of the otherwise cylindrical slidable end-piece 222, which is visible in Figure 6. The groove and tooth engage to define a connection so that the metering cursor 219' moves with the end-piece 222. To ensure a more stable connection, the two elements can also be glued or soldered.

In an alternative embodiment not visible in the attached Figures, the groove may not be a pass-through groove, being only present on the inner wall. Therefore, the groove is not visible from the outside. A hole can be added on the sliding body 2121 to allow gluing/welding of the cursor.

The internal metering cursor 219' further features a lateral protrusion 2191 which extends at least partly through the slit 220 and which exhibits an index 2192 for visual reading of the element's position of on an underlying metered scale.

The internal metering cursor 219' comprises a copper layer 2193, gold-plated to avoid corrosion issues, which is fixed on the underside of the sled-shaped body. The copper layer 2193 is meant to amplify the antenna gain, thereby increasing the sensitivity of the metering device.

The inductive position sensor 214' further comprises a sensing strip 2142, which is obtained as a flexible printed circuit board in a *per se* known manner.

**Figure 6** shows a cross-section of the external fixation strut 200' which shows further details of the inductive position sensor 214' and of its sensing strip 2142.

The sensing strip 2142 is attached over an elongated support plate 2143 made of ferromagnetic material, for instance mu-metal. The ferrite plate 2143 is meant to shield the sensor from external interferences. The top face of the sensing strip 2142 faces the internal metering cursor 219' and the ferrite plate 2143 backs the sensing strip 2142 on the other side.

In device manufacturing, the sensing strip 2142 can be glued to the ferrite plate 2143 via a double-sided adhesive strip which is provided on the sensing strip 2142. Then, the combined plate-and-strip are inserted within the sliding body 2121 through the slit 220. As an alternative, the ferromagnetic plate is directly provided as a last layer of the sensing strip.

The position sensor 214' is preferably an absolute sensor.

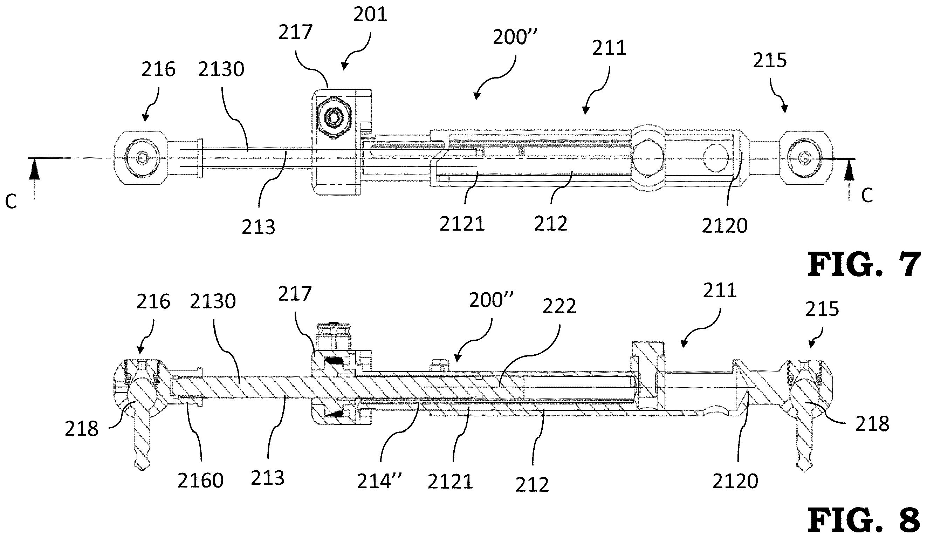

**Figure 7** shows a schematic side view of a third embodiment of the external fixation strut 200" according to the invention.

The third embodiment is identical to the previous ones except for the position sensor 214", which is based on a capacitive technology.

**Figure 8** shows a cross-sectional view of the device of figure 7.

The position sensor 214" is glued on an internal surface of the sliding body 2121, and it measures the relative position of the end-piece 222.

The position sensor 214 is therefore housed within the casing 217, solidly attached to it and it extends along the longitudinal inner length of the first shaft 212.

The position sensor 214 detects an absolute position of the end-piece 222 of the second shaft 213, returning a measurement value which can be employed to assess the relative position of the second shaft 213 with respect to the first shaft 212.

**Figure 9** shows a schematic perspective view of the external fixation strut 200" according to the third embodiment of the invention, with no further elements disclosed.

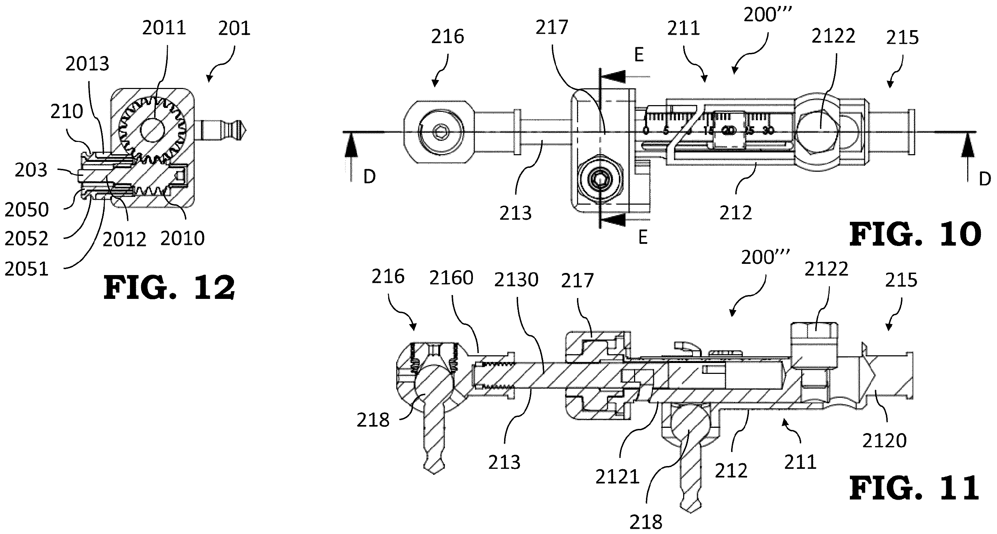

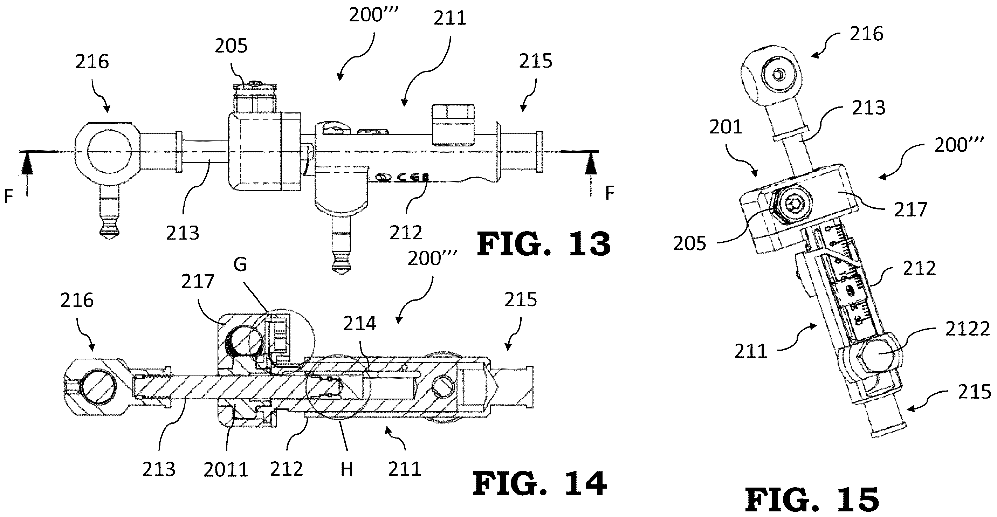

**Figures 10-15** refer to fourth embodiment of an external fixation strut 200"', which is shorter in size with respect to the long external fixation strut 200' according to the first embodiment previously discussed.

In such an alternative embodiment, the external fixation strut 200‴ essentially has the same components and main features of the longer embodiments, which are previously described. Such components and features are therefore indicated in the figures by reference numerals previously used, and they are not described anew in the following paragraphs.

The main structural difference with respect to the longer version is that here the two attachment points defined by the ball-joints 218 are set closer to each other, thanks to the fact that the ball-joint of the first shaft 212 is not placed at the end 215 thereof, but rather at an opposite end of the hollow main body 2120 of the first shaft 215.

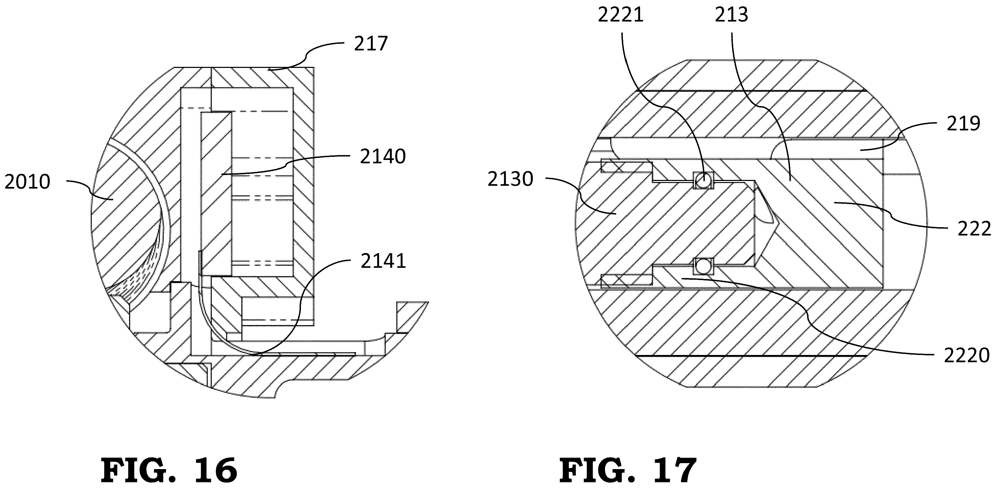

**Figure 16** shows a detail in the construction of the previously introduced position sensor 214.

A flexible signal line 2141 of the position sensor, made in form of a flat cable, connects an electronic board 2140 to the body of the sensor 214, which extends in the vicinity of the sensed end-piece 222.

The electronic board 2140 is meant for sensor powering and data transmission and is meant to connect to a programmable tool 100 via the connecting system which is described in the following.

It is observed that the flexible signal line 2141 has at least one curvature to connect the rotatable adjustment mechanism's housing to the rest of the shaft. Said curvature is designed such that the radius is kept above a threshold value to avoid damaging the signal line during the manufacturing process.

The same construction of the electronic board 2140 with flexible signal line 2141 is also preferably adopted in the position sensors 214', 214" of the second and third embodiments.

**Figure 17** shows a detail in the construction of the previously introduced end-piece 222.

As seen in the Figure, the end of the shank 2130 is inserted in a sleeve 2220 of the end-piece 222 in a rotatable manner, through an interposed ring 2221. The ring 2221 allows the end-piece 222 and thus the cursor 219 to translate along the longitudinal axis of the strut 200"' while the shank 2130 rotates.

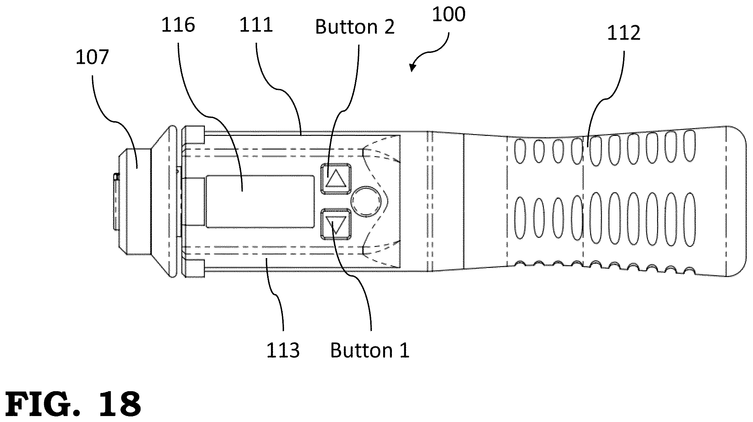

**Figure 18** shows a top view of a programmable tool 100 according to the present disclosure, which is in the form of an electric wrench.

The wrench comprises a rigid external casing 111.

In the depicted embodiment, the casing 111 has a substantially cylindrical shape. However, the shape of the housing 111 may be any shape and size convenient for use.

In the depicted embodiment, the casing 111 defines a handpiece having a distal gripping portion 112 with an ergonomic handle followed by a proximal interface portion 113.

The housing may have a variety of connection ports, connectors, display and controls.

In the depicted embodiment, the interface portion comprises a first and a second pushbutton, identified as Button 1 and Button 2, and a display 116, and further has an annular LED indicator which can be lit in various colors and in a steady or pulsing mode in order to signal a plurality of device statuses to the user.

In a preferred embodiment, the annular LED indicator may have up to four lighted states, respectively signamgling: a call to action by the user, an ongoing operation by the wrench, successful or unsuccessful completion of a wrench operation.

The LED indicator is preferably covered by a transparent light guide which is integrated into the casing 111.

The device may further comprise a buzzer or any other audio device.

The device further comprises a front muzzle 107 ahead of the interface portion 113, which is arranged to connect to the torque input port of the rotatable adjustment mechanism 201 of the external fixation struts 200, 200'.

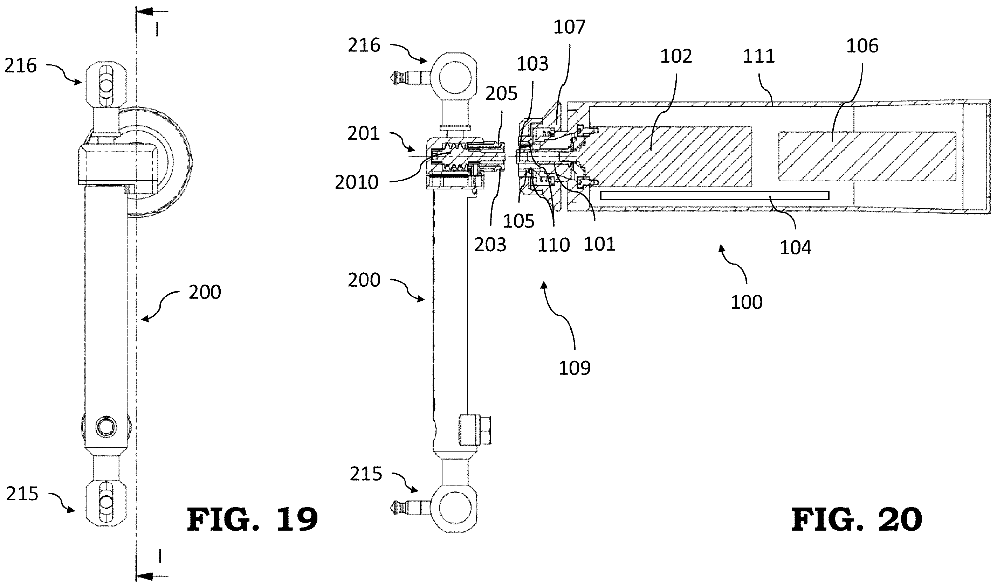

**Figure 19** shows a side view of the programmable tool 100 about to be coupled with an external fixation strut 200.

**Figure 20** is a cross-sectional view of the two devices of figure 19, and schematically shows the main components inside the casing 111 of the programmable tool 100.

The programmable tool 100 comprises power source 106 in the form of a rechargeable battery, an electric motor 102 which preferably comprises a gearbox in-line with a coaxial output shaft 101, and a controller 104 in the form of a PCB.

The battery 106 is preferably recharged wirelessly, through inductive technology, to minimize electrical hazards for the user.

The programmable tool 100 may also comprise an internal memory, which can be conveniently used for storing a patient's prescription and an actual length of a plurality of struts making up an external fixator to be adjusted by the tool 100.

The programmable tool 100 has means of communicating with an external data source, in the form of data ports and/or wireless connectivity. Preferably, the programmable tool as a SIM housing (not shown) which is meant to provide internet connectivity to the device.

The controller 104 of the programmable tool 100 is adapted to communicate with an external portal, directly or via a portable electronic device such as a smartphone, to retrieve a surgeon's prescription and update a status comprising at least the length of the several external fixation struts 200, 200', 200", 200‴ of an external fixator.

The controller 104 is connected to the pushbuttons 114, 115, display 116 and annular LED indicator in order to read user's commands, communicate status updates, or guide the user throughout a process of adjusting all of the struts 200, 200', 200", 200‴ according to a given prescription.

The muzzle 107 of the programmable tool 100 houses the output shaft 101, which culminates with a first coupling portion 103 in the form of a wrench socket, in particular a hex socket.

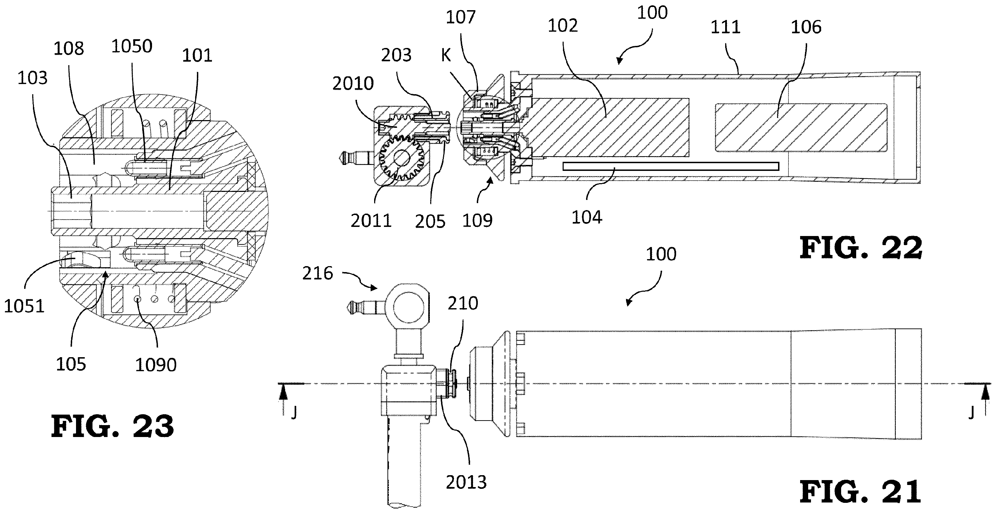

The muzzle further comprises a locking mechanism 109 operable to secure and selectively release the engagement of the first coupling portion 103 with the second coupling portion 203. When the locking mechanism 109 is engaged the protruding head 2013 of the rotatable adjustment mechanism 201 is housed within an annular recess 108 of the muzzle 107, best shown in Figure 23.

The locking mechanism 109 is defined by the body of the muzzle 107, which may be retracted towards the programmable tool 100 housing 111 by pressing it against elastic means, which may be in the form of a spring 1090 best shown in Figure 23. The muzzle 107 internally has latching elements 110, which may be in the form of rollers moving along oblique paths, said roller being externally biased by the action of the spring 1090 in order to engage the first coupling portion 103.

Around said first coupling portion 103 is provided a first connecting portion 105, which is meant to electrically connect with a second connecting portion 205 of the strut 200, 200'.

When the locking mechanism 109 is engaged, the first connecting portion 105 is connected to the second connecting portion 205 of the strut 200, 200' and data connection is provided which allows the controller 104 to retrieve data from sensor 214.

The electric connection further ensures powering of the sensor 214 from the power source 106.

**Figure 21** shows a further side view of the programmable tool 100 about to be coupled with an external fixation strut 200.

**Figure 22** is a cross-sectional view of the two devices of figure 18.

**Figure 23** is an enlargement of figure 19, which shows a detail of the first connecting portion 105.

The first connecting portion 105 comprises a plurality of inner connector 1050 at the bottom of the annular recess 108, which are preferably in the form of pogo-pins, meant to connect a first electric pole.

Further, the first connecting portion 105 comprises a plurality of outer connectors 1051, preferably in the form of clip, tape spring or leaf spring connectors, projecting from the outer lateral surface of said annular recess 108. Said outer connectors 1051 are meant to connect a second electric pole.

In use, the inner connectors 1050 contact an inner surface 2050 of the protruding head 2013 outer sleeve, while the outer connectors 1051 contact an outer surface 2051 of the same sleeve. As best seen in Figure 12, said inner surface 2050 and outer surface 2051 are separated by a dielectric layer 2052.

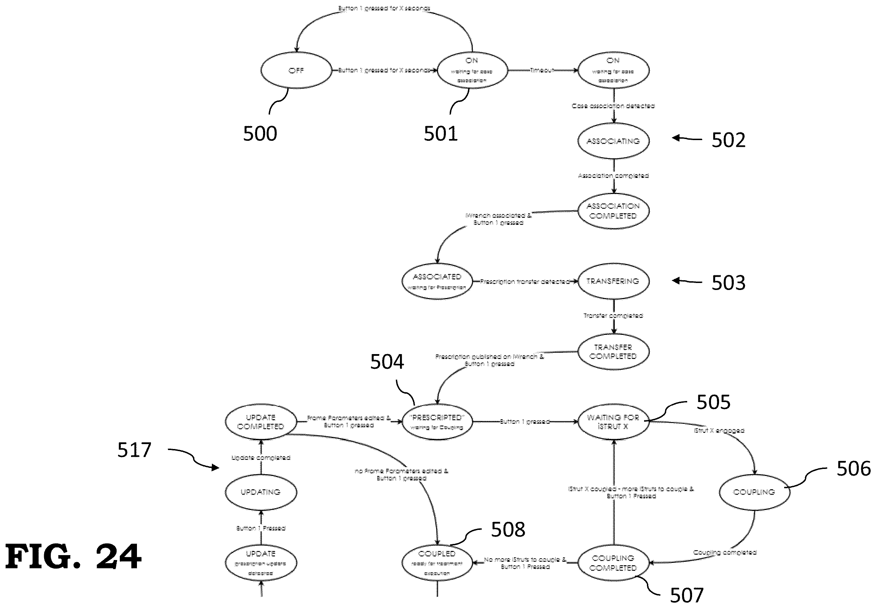

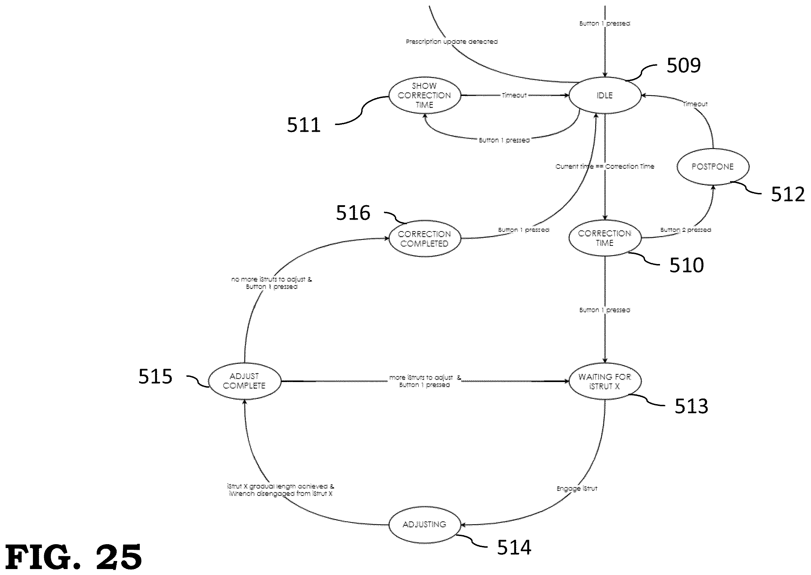

**Figure 24** **and** **25** show a top and a bottom part showing a flow diagram of an operational method of the programmable tool 100.

The device can be turned from an off state 500 to an on state 501 by the user, for instance by pressing Button 1 or Button 2 for a given time.

A similar user action may be demanded to return the device to the off state 500.

After a given time from being turned on, the device will be associated to a specific case 502.

Then, after a command by the user, the device will connect to the internet and retrieve from a dedicated portal the prescription for the patient's case 503.

After the prescription has been uploaded to the internal memory, the device is in a general waiting state 504 which may be signaled to the user via the display 116 and/or the annular LED indicator.

Upon a user command, for instance the pressure of Button 1, the device is in a coupling waiting state 505 and it will signal through the display 116 that it is ready to connect with a given strut.

In coupling waiting state 505, the user is expected to mechanically couple the device to a strut 200, 200' which, as previously discussed, also results in a data connection.

When the data connection is performed 507, the device advises the user which may then couple the other struts 200, 200' in a similar way.

Once all struts 200, 200' have been correctly coupled, the device enters a coupled waiting state 508.

Then, upon a command by the user, the device enters an idle state 509 ready to signal when a prescribed correction time 510 is reached. In such a state, the user can interrogate the device - for instance, by pressing Button 1 - which will then display the next correction time 511.

When the correction time 510 is eventually reached, the user may be advised via an audio and/or a visual signal that he/she is required to take action.

The user will then be granted the choice of either performing or postponing 512 the adjustment of the struts. Button 1 and Button 2 can be alternately employed to discriminate between the two choices.

If the user decides to perform the correction, the device will then guide the user toward engaging the struts 200, 200' to be adjusted 513. Once a strut 200, 200' is coupled, the device will automatically perform the adjustment in an adjustment step 514, and thereafter signal to the user that the adjustment is complete 515.

If another strut is to be adjusted, steps 513-515 are repeated; otherwise, the device signals to the user that the correction is completed 516, and returns to the idle state 509.

From the idle state 509, the device is also able to update the prescription 517 whenever a prescription update is detected.

It will be understood that particular embodiments described herein are shown by way of illustration and not as limitations of the disclosure. The principal features of this disclosure can be employed in various embodiments without departing from the scope of the disclosure. Those skilled in the art will recognize, or be able to ascertain using no more than routine experimentation, numerous equivalents to the specific devices and procedures described herein.

All publications and patent applications mentioned in the specification are indicative of the level of skill of those skilled in the art to which this disclosure pertains.

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

## Claims

1. An external fixation strut (200, 200', 200", 200"'), comprising:
an elongated body (211) comprising at least a first shaft (212) and a second shaft (213) moveable with respect to each other to modify the length of said elongated body (211);
at least a sensor (214, 214', 214") integrated in the external fixation strut (200, 200', 200", 200"') and adapted to perform at least a measurement which is indicative of the length of the elongated body (211);
wherein said sensor (214, 214', 214") is configured to use either an inductive or a capacitive technology to determine the position of a movable element;
**characterized in that** said sensor (214, 214', 214") is configured to communicate with a programmable tool (100) for lengthening/shortening the external fixation strut (200, 200', 200", 200"') through a data port which is engageable by said programmable tool (100);
wherein said movable element is an end-piece (222) of the second shaft (213) and/or a metering cursor (219, 219') which is configured to translate with said end-piece (222) with respect to the second shaft (213);
wherein said end-piece (222) is slidable within a tubular body (217a) of the first shaft (211);
wherein the sensor comprises a sensing strip (2142) arranged within the tubular body (217a) of the first shaft (211);
wherein said sensing strip (2142) is a flexible printed circuit board and it is attached on top of a rigid support plate (2143);
wherein said rigid support plate (2143) is made of ferromagnetic material and at least partly shields the sensing strip (2142) from external noise;
wherein a longitudinal slit (220) is provided at least internally along the tubular body (217a) of the first shaft (211), so that the sensing strip (2142) and the rigid support plate (2143) can be inserted through said longitudinal slit (220) in a mounting phase.

2. The external fixation strut (200"') of claim 1, wherein at least a predominant part of said metering cursor (219') is housed within the tubular body (217a) of the first shaft (211).

3. The external fixation strut (200‴) of claim 2, wherein said metering cursor (219') is at least partially housed within a longitudinal slit (220) of the tubular body (217a) of the first shaft (211), said longitudinal slit (220) extending in a longitudinal direction of the strut (200"').

4. The external fixation strut (200‴) of claim 3, wherein said metering cursor (219') engages with the end-piece (222) via a groove and tooth arrangement which extends in a transverse direction orthogonal to the longitudinal direction of the strut (200‴), so that the metering cursor (219') can be mounted by inserting it through the longitudinal slit (220).

5. The external fixation strut (200, 200', 200", 200‴) of claim 1, wherein said second shaft (213) comprises a casing (217) having a tubular body (217a) and a protruding portion (217b) extending beyond the diameter of said tubular body (217a), said protruding portion (217b) housing an electronic board (2140) for sensor powering and data transmission which is not aligned with the sensing strip (2143), the electronic board (2140) being connected to the sensing strip (2142) through a flexible signal line (2141).

6. The external fixation strut (200, 200', 200", 200"') of claim 1, wherein said sensor (214, 214', 214") is powered through said port.

7. The external fixation strut (200, 200', 200", 200"') of claim 1, further comprising a rotatable adjustment mechanism (201) for moving the second shaft (213) with respect to the first shaft (212) thus modifying the length of the elongated body (211); a second coupling portion (203) of the rotatable adjustment mechanism (201) adapted to releasably engage with a first coupling portion (103) of the programmable tool (100) to enable torque transmission; wherein said port is defined by a second connecting portion (205) of the rotatable adjustment mechanism (201) which is in electrical communication with a first connecting portion (105) of the programmable tool (100) when the first coupling portion (103) is engaged with the second coupling portion (203), and which enables transmission of data from the sensor (214, 214', 214") to the programmable tool (100).

8. A medical kit comprising at least one external fixation strut (200, 200', 200", 200"') according to claim 1 and a programmable tool (100) operable to adjust said external fixation strut (200, 200', 200", 200‴) and comprising:
an output shaft (101);
a motor (102) operable to rotate said output shaft (101);
a first coupling portion (103) solidly attached to said output shaft (101) and adapted to releasably engage with a corresponding second coupling portion (203) of a rotatable adjustment mechanism (201) of the external fixation strut (200, 200', 200", 200"'), thus enabling torque transmission from the motor (102) to the rotatable adjustment mechanism (201);
a controller (104) configured for driving said motor (102) according to a prescription comprising instructions for adjusting the external fixation strut (200, 200', 200", 200‴);
a first connecting portion (105) in electrical communication with said controller (104) and adapted to electrically connect with a second connecting portion (205) of the rotatable adjustment mechanism (201) when the first coupling portion (103) is engaged with the second coupling portion (203), thus enabling data transmission from said sensor (214, 214', 214") to the controller (104).

## Patentansprüche

1. Externe Befestigungsstütze (200, 200', 200", 200‴), die Folgendes umfasst:
einen langgestreckten Körper (211), der mindestens eine erste Welle (212) und eine zweite Welle (213) umfasst, die in Bezug aufeinander beweglich sind, um die Länge des langgestreckten Körpers (211) zu ändern; und
mindestens einen Sensor (214, 214', 214"), der in die externe Befestigungsstütze (200, 200', 200", 200‴) integriert ist und ausgelegt ist, mindestens eine Messung durchzuführen, die die Länge des langgestreckten Körpers (211) angibt;
wobei der Sensor (214, 214', 214") konfiguriert ist, entweder eine induktive oder eine kapazitive Technologie zu verwenden, um die Stellung eines beweglichen Elements zu bestimmen;
**dadurch gekennzeichnet, dass** der Sensor (214, 214', 214") konfiguriert ist, mit einem programmierbaren Werkzeug (100) zum Verlängern/Verkürzen der externen Befestigungsstütze (200, 200', 200", 200‴) über einen Datenanschluss zu kommunizieren, mit dem das programmierbare Werkzeug (100) in Eingriff gelangen kann;
wobei das bewegliche Element ein Endstück (222) der zweiten Welle (213) und/oder ein Messcursor (219, 219'), der konfiguriert ist, mit den Endstück (222) in Bezug auf die zweite Welle (213) versetzt zu werden, ist;
das Endstück (222) in einem rohrförmigen Körper (217a) der ersten Welle (211) schiebbar ist;
der Sensor einen Erfassungsstreifen (2142) umfasst, der im rohrförmigen Körper (217a) der ersten Welle (211) angeordnet ist;
der Erfassungsstreifen (2142) eine flexible gedruckte Leiterplatte ist und auf einer starren Trägerplatte (2143) angebracht ist;
die starre Trägerplatte (2143) aus einem ferromagnetischem Material hergestellt ist und den Erfassungsstreifen (2142) von externem Rauschen mindestens teilweise abschirmt und
ein Längsschlitz (220) mindestens intern entlang des rohrförmigen Körpers (217a) der ersten Welle (211) vorgesehen ist, derart, dass in einer Anbringungsphase der Erfassungsstreifen (2142) und die starre Trägerplatte (2143) durch den Längsschlitz (220) eingesetzt werden können.

2. Externe Befestigungsstütze (200"') nach Anspruch 1, wobei mindestens ein überwiegender Teil des Messcursors (219') in den rohrförmigen Körper (217a) der ersten Welle (211) aufgenommen ist.

3. Externe Befestigungsstütze (200‴) nach Anspruch 2, wobei der Messcursor (219') in einen Längsschlitz (220) des rohrförmigen Körpers (217a) der ersten Welle (211) mindestens teilweise aufgenommen ist und der Längsschlitz (220) sich in einer Längsrichtung des Federbeins (200‴) erstreckt.

4. Externe Befestigungsstütze (200‴) nach Anspruch 3, wobei der Messcursor (219') mit dem Endstück (222) mittels einer Nut- und Zahnanordnung in Eingriff ist, die sich in einer Querrichtung senkrecht zur Längsrichtung des Federbeins (200‴) erstreckt, derart, dass der Messcursor (219') durch sein Einsetzen durch den Längsschlitz (220) montiert werden kann.

5. Externe Befestigungsstütze (200, 200', 200", 200‴) nach Anspruch 1, wobei die zweite Welle (213) ein Gehäuse (217) umfasst, das einen rohrförmigen Körper (217a) und einen Vorsprungsabschnitt (217b), der sich über den Durchmesser des rohrförmigen Körpers (217a) hinaus erstreckt, aufweist, wobei der Vorsprungsabschnitt (217b) eine elektronische Platine (2140) zur Sensorenergieversorgung und Datenübertragung aufnimmt, die nicht auf den Erfassungsstreifen (2143) ausgerichtet ist, und die elektronische Platine (2140) mit dem Erfassungsstreifen (2142) über eine flexible Signalleitung (2141) verbunden ist.

6. Externe Befestigungsstütze (200, 200', 200", 200‴) nach Anspruch 1, wobei der Sensor (214, 214', 214") über den Anschluss mit Energie versorgt wird.

7. Externe Befestigungsstütze (200, 200', 200", 200‴) nach Anspruch 1, die ferner einen drehbaren Einstellmechanismus (201) zum Bewegen der zweiten Welle (213) in Bezug auf die erste Welle (212), wodurch die Länge des langgestreckten Körpers (211) geändert wird, umfasst; wobei ein zweiter Kopplungsabschnitt (203) des drehbaren Einstellmechanismus (201) ausgelegt ist, mit einem ersten Kopplungsabschnitt (103) des programmierbaren Werkzeugs (100) lösbar in Eingriff zu sein, um eine Drehmomentübertragung zu ermöglichen; und der Anschluss durch einen zweiten Verbindungsabschnitt (205) des drehbaren Einstellmechanismus (201) definiert ist, der mit einem ersten Verbindungsabschnitt (105) des programmierbaren Werkzeugs (100) in elektrischer Kommunikation ist, wenn der erste Kopplungsabschnitt (103) mit dem zweiten Kopplungsabschnitt (203) in Eingriff ist, und der eine Übertragung von Daten vom Sensor (214, 214', 214") zum programmierbaren Werkzeug (100) ermöglicht.

8. Medizinisches Kit bestehend aus mindestens einer externen Befestigungsstütze (200, 200', 200", 200‴) nach Anspruch 1 und einem programmierbares Werkzeug (100), das betreibbar ist, die externe Befestigungsstütze (200, 200', 200", 200‴) einzustellen, und Folgendes umfasst:
eine Antriebswelle (101);
einen Motor (102), der betreibbar ist, die Antriebswelle (101) zu drehen;
einen ersten Kopplungsabschnitt (103), der an der Antriebswelle (101) fest angebracht ist und ausgelegt ist, mit einem entsprechenden zweiten Kopplungsabschnitt (203) eines drehbaren Einstellmechanismus (201) der externen Befestigungsstütze (200, 200', 200", 200‴) lösbar in Eingriff zu sein, wodurch eine Drehmomentübertragung vom Motor (102) zum drehbaren Einstellmechanismus (201) ermöglicht wird;
eine Steuereinheit (104), die konfiguriert ist zum Ansteuern des Motors (102) gemäß einer Verschreibung, die Anweisungen zum Einstellen der externen Befestigungsstütze (200, 200', 200", 200‴) umfasst; und
einen ersten Verbindungsabschnitt (105) in elektrischer Kommunikation mit der Steuereinheit (104), der ausgelegt ist, eine elektrische Verbindung mit einem zweiten Verbindungsabschnitt (205) des drehbaren Einstellmechanismus (201) herzustellen, wenn der erste Kopplungsabschnitt (103) mit dem zweiten Kopplungsabschnitt (203) in Eingriff ist, wodurch eine Datenübertragung vom Sensor (214, 214', 214") zur Steuereinheit (104) ermöglicht wird.

## Revendications

1. Tige de fixation externe (200, 200', 200", 200"'), comprenant:
un corps allongé (211) comprenant au moins un premier arbre (212) et un deuxième arbre (213) mobiles l'un par rapport à l'autre pour modifier la longueur dudit corps allongé (211);
au moins un capteur (214, 214', 214") intégré dans la tige de fixation externe (200, 200', 200", 200"') et adapté pour effectuer au moins une mesure qui est indicative de la longueur du corps allongé (211);
ledit capteur (214, 214', 214") étant configuré pour utiliser une technologie inductive ou capacitive pour déterminer la position d'un élément mobile;
**caractérisée en ce que** ledit capteur (214, 214', 214") est configuré pour communiquer avec un outil programmable (100) pour allonger/raccourcir la tige de fixation externe (200, 200', 200", 200"') via un port de donnée qui est engageable par ledit outil programmable (100);
ledit élément mobile étant un embout (222) du deuxième arbre (213) et/ou un curseur de mesure (219, 219') qui est configuré pour translater avec ledit embout (222) par rapport au deuxième arbre (213);
ledit embout (222) pouvant coulisser à l'intérieur d'un corps tubulaire (217a) du premier arbre (211);
le capteur comprenant une bande de détection (2142) arrangée à l'intérieur du corps tubulaire (217a) du premier arbre (211);
ladite bande de détection (2142) étant une carte de circuit imprimé flexible et étant fixée sur une plaque de support rigide (2143);
ladite plaque de support rigide (2143) étant composée d'un matériau ferromagnétique et protégeant au moins partiellement la bande de détection (2142) du bruit extérieur;
dans laquelle une fente longitudinale (220) est prévue au moins intérieurement le long du corps tubulaire (217a) du premier arbre (211), de sorte que la bande de détection (2142) et la plaque de support rigide (2143) puissent être insérées à travers ladite fente longitudinale (220) dans une étape de montage.

2. Tige de fixation externe (200"') selon la revendication 1, dans laquelle au moins une partie prédominante dudit curseur de mesure (219') est logée à l'intérieur du corps tubulaire (217a) du premier arbre (211).

3. Tige de fixation externe (200"') selon la revendication 2, dans laquelle ledit curseur de mesure (219') est logé au moins partiellement à l'intérieur d'une fente longitudinale (220) du corps tubulaire (217a) du premier arbre (211), ladite fente longitudinale (220) s'étendant dans une direction longitudinale de la tige (200"').

4. Tige de fixation externe (200"') selon la revendication 3, dans laquelle ledit curseur de mesure (219') s'engage avec l'embout (222) au moyen d'un arrangement de rainure et dent s'étendant dans une direction transversale orthogonale à la direction longitudinale de la tige (200"'), de sorte que le curseur de mesure (219') puisse être monté en l'insérant à travers la fente longitudinale (220).

5. Tige de fixation externe (200, 200', 200", 200"') selon la revendication 1, dans laquelle ledit deuxième arbre (213) comprend un boîtier (217) ayant un corps tubulaire (217a) et une portion en saillie (217b) s'étendant au-delà du diamètre dudit corps tubulaire (217a), ladite portion en saillie (217b) logeant une carte électronique (2140) pour l'alimentation du capteur et la transmission de données qui n'est pas alignée avec la bande de détection (2143), la carte électronique (2140) étant connectée à la bande de détection (2142) par une ligne de signal flexible (2141).

6. Tige de fixation externe (200, 200', 200", 200‴) selon la revendication 1, dans laquelle ledit capteur (214, 214', 214") est alimenté par ledit port.

7. Tige de fixation externe (200, 200', 200", 200‴) selon la revendication 1, comprenant en outre un mécanisme d'ajustement rotatif (201) pour déplacer le deuxième arbre (213) par rapport au premier arbre (212) modifiant ainsi la longueur du corps allongé (211); une deuxième portion d'accouplement (203) du mécanisme d'ajustement rotatif (201) adaptée pour s'engager de manière amovible avec une première portion d'accouplement (103) de l'outil programmable (100) pour permettre la transmission du couple; dans laquelle ledit port est défini par une deuxième portion de connexion (205) du mécanisme d'ajustement rotatif (201) qui est en communication électrique avec une première portion de connexion (105) de l'outil programmable (100) lorsque la première portion d'accouplement (103) est en prise avec la deuxième portion d'accouplement (203), et qui permet la transmission de données à partir du capteur (214, 214', 214") à l'outil programmable (100).

8. Kit médical comprenant au moins une tige de fixation externe (200, 200', 200", 200"') selon la revendication 1 et un outil programmable (100) utilisable pour ajuster ladite tige de fixation externe (200, 200', 200", 200"') et comprenant:
un arbre de sortie (101);
un moteur (102) utilisable pour faire tourner ledit arbre de sortie (101);
une première portion d'accouplement (103) fixée solidement audit arbre de sortie (101) et adaptée pour s'engager de manière amovible avec une deuxième portion d'accouplement (203) correspondante d'un mécanisme d'ajustement rotatif (201) de la tige de fixation externe (200, 200', 200", 200"'), permettant ainsi la transmission du couple à partir du moteur (102) au mécanisme d'ajustement rotatif (201);
un contrôleur (104) configuré pour entraîner ledit moteur (102) selon une prescription comprenant des instructions pour ajuster la tige de fixation externe (200, 200', 200", 200"');
une première portion de connexion (105) en communication électrique avec ledit contrôleur (104) et adaptée pour se connecter électriquement à une deuxième portion de connexion (205) du mécanisme d'ajustement rotatif (201) lorsque la première portion d'accouplement (103) est en prise avec la deuxième portion d'accouplement (203), permettant ainsi la transmission de données à partir dudit capteur (214, 214', 214") au contrôleur (104).
